# EUROPEAN PATENT APPLICATION

(11) **EP 4 137 796 A1**
(43) Date of publication of application: **22.02.2023**
(21) Application number: 21759520.6
(22) Date of filing: 20.02.2021
(51) Int. Cl.: G01N 21/00, G01N 21/64, G01N 33/53

(54) **DETECTION PARTICLE SUITABLE FOR MULTIPLEX DETECTION OF BIOMOLECULES, AND PREPARATION METHOD THEREFOR AND APPLICATION THEREOF**

(30) Priority: 25.02.2020 CN 202010129075
(71) Applicant: Shanghai Jiao Tong University, Shanghai 200240 (CN); Shanghai Maijing Nano Technology Co., Ltd., Shanghai 200030 (CN)
(72) Inventor: GUO, Qingsheng, Shanghai 200240 (CN); XU, Hong, Shanghai 200240 (CN); GU, Hongchen, Shanghai 200240 (CN); FENG, Zuying, Shanghai 200240 (CN)
(74) Representative: Canzler & Bergmeier Patentanwälte Partnerschaft mbB
(86) International application number: PCT/CN2021/077031
(87) International publication number: WO 2021/169866

(57) **Abstract**

A detection particle suitable for multiplex detection of biomolecules, comprising: a microcarrier having a coding function, the microcarrier being connected to detection microparticles, and the detection microparticles being suitable for executing light initiated chemiluminescence detection. A multifunctional detection particle that integrates functions of code discrimination, magnetic separation and light initiated chemiluminescence detection is prepared for the first time. An imaging detection and analysis device used for the multifunctional detection particle is built for the first time. The provided detection particle suitable for multiplex detection of biomolecules achieves a highly sensitive, fast and wash-free multiplex detection method, greatly expands the number of biomolecules that can be detected in a single reaction, and achieves removal of an unreacted signal labelled molecule without washing in the whole process, so that the experimental steps are simplified and the detection efficiency is improved. The method is suitable for detection of proteins, nucleic acids and small molecules.

## Description

### FIELD OF DISCLOSURE

The present invention relates to the field of biological detection, particularly to a detection particle suitable for multiplex detection of biomolecules, a method for preparing the same, and the use thereof.

### DESCRIPTION OF RELATED ARTS

Biomolecule detection, especially immune detection based on antigen-antibody recognition and nucleic acid detection based on complementary base pairing, plays an important role in disease diagnosis, drug development, basic biological science research, and the like. Enzyme-linked immunosorbent assay (ELISA) has become the gold standard technology for immune detection due to its high sensitivity and specificity. However, there are still two major challenges for ELISA. On the one hand, a typical ELISA detection process requires repeated separation and washing to remove unbound molecules which may bring background signals. This makes the analysis complex and time-consuming, and multi-step operations can lead to cumulative errors. Therefore, the development of a convenient and easy-to-use immune detection method will better meet the needs of on-site rapid detection. Light-initiated chemiluminescent technology employs the chemiluminescent signal generated by the transfer of singlet oxygen between two particles after light excitation to detect the analyte, thus avoiding the repeated washing required by traditional ELISA. However, light-initiated chemiluminescent technology lacks the ability of multiplex detection based on a single reaction. On the other hand, due to that multiple markers are associated with one disease, a more accurate, sensitive, and effective diagnosis can be achieved through single-reaction multiplex detection of these marker molecules. Encoding microbead technology uses microbeads with unique encoding characteristics as reaction units to identify specific target molecules, which can realize simultaneous detection of multiple substances to be tested in one reaction tube. However, repeated separation and complicated washing like ELISA are required, therefore, it is difficult to meet the needs of application scenarios such as on-site rapid detection.

### SUMMARY OF THE PRESENT INVENTION

The present invention provides a detection particle suitable for multiplex detection of biomolecules, a method for preparing the same, and the use thereof.

A first aspect of the present invention provides a detection particle suitable for multiplex detection of biomolecules, which includes a microcarrier with an encoding function, and detection microparticles connected to the microcarrier, where the detection microparticles are suitable for light-initiated chemiluminescent detection.

A second aspect of the present invention provides a biomolecule detection system for simultaneous detection of multiple biomolecules, which includes at least a plurality of the aforementioned detection particles suitable for multiplex detection of biomolecules, and matching microparticles, where the matching microparticles refer to light-initiated chemiluminescent microparticles matched with the detection microparticles in the detection particles.

A third aspect of the present invention provides a method for preparing the detection particle suitable for multiplex detection of biomolecules, which includes the following operations:

Mixing the microcarrier with the encoding function and a dispersion containing the detection microparticles, performing a reaction in a dark room, to obtain the detection particle suitable for multiplex detection of biomolecules.

A fourth aspect of the present invention provides the use of the aforementioned detection particle suitable for multiplex detection of biomolecules or the aforementioned biomolecule detection system in biomolecule detection.

A fifth aspect of the present invention provides a biomolecule detection method, which includes the following operations:
(1) mixing the detection particle suitable for multiplex detection of biomolecules with a sample solution to be tested, and incubating;
(2) adding the matching microparticles, and incubating;
(3) placing the mixture obtained in step (2) in a fluorescence microscope with a camera or photographic function to perform detection, and exciting the signal of the microcarrier with the encoding function and the light-initiated chemiluminescent signal;
(4) collecting the signal of the microcarrier with the encoding function and the light-initiated chemiluminescent signal, respectively, and matching the image obtained after excitation of the signal of the microcarrier with the encoding function with the image obtained after excitation of the light-initiated chemiluminescent signal.

The present invention provides a detection particle suitable for multiplex detection of biomolecules, a method for preparing the same, and the use thereof, which have the following advantages:

A multifunctional detection particle integrating encoding identification, magnetic separation, and light-initiated chemiluminescent detection functions was firstly prepared in the present disclosure. And an imaging detection and analysis device for the multifunctional detection particle was firstly provided. The provided detection particle suitable for multiplex detection of biomolecules can realize a highly sensitive, fast, and washing-free multiplex detection method, which greatly expands the kind of biomolecules that can be detected by a single reaction, and can remove unreacted signal labeled molecules without washing during the whole process, thus simplifying experimental steps, and improving detection efficiency. The method is suitable for the detection of proteins, nucleic acids, and small molecules.

### BRIEF DESCRIPTION OF THE DRAWINGS

FIG. 1 is a schematic diagram of a preparation process for a detection particle without biocapture substance in an embodiment of the present invention.
FIG. 1-1 is a schematic diagram showing the bind of a biomolecule detection system in an embodiment of the present invention to a molecule to be detected.
FIG. 2 is a schematic diagram of scanning electron microscopic images for a quantum dot-encoded microcarrier and the detection particle without biocapture substance in an embodiment of the present invention.
FIG. 3 is a schematic diagram of scanning electron microscopic images for a microcarrier covered by a polyelectrolyte shell layer and filled with a fluorescent dye and the detection particle without biocapture substance in an embodiment of the present invention.
FIG. 4 is a schematic diagram of scanning electron microscopic images for a detection particle prepared from an encoded microcarrier covered by multiple polyelectrolyte shell layers and a detection particle prepared from an encoded microcarrier not covered by a polyelectrolyte shell layer in an embodiment of the present invention.
FIG.5 is a schematic diagram of the principle of the present invention and a flow diagram of a sample detected under a "washing-free" strategy in an embodiment.
FIG. 6 is a standard curve of IFN-γ antigen standard detection under a "washing-free" strategy by using the detection particle prepared from the microcarrier filled with fluorescent dye in an embodiment of the present invention.
FIG. 7 is a standard curve of multiplex antigen standard detection under the "washing-free" strategy by using the detection particle prepared from the quantum dot-encoded microcarrier in an embodiment of the present invention.
FIG. 8 is a standard curve of multiplex antigen standard detection under a "washing" strategy by using the detection particle prepared from the quantum dot-encoded microcarrier in an embodiment of the present invention.

### DETAILED DESCRIPTION OF THE PREFERRED EMBODIMENTS

Light-initiated chemiluminescent technology (LICA) consists of a donor ball (DB) and an acceptor ball (AB). The DB is doped with a photosensitizer, which can produce singlet oxygen after being excited by light. The AB is doped with thioxene and lanthanide complex, where thioxene converts the energy of singlet oxygen into emitting light with 360 nm, and excites the lanthanide complex to produce fluorescence. After being irradiated by a 680 nm laser, DB can activate the oxygen in the surrounding environment and convert them into singlet oxygen whose survival time only lasts 4 microseconds. The short survival time determines that the spreading diameter of ionic oxygen is very small (about 200 nm). When there is a molecule to be tested, it forms an immune complex with AB of a conjugated capture antibody and DB of a conjugated detection antibody through a double-antibody sandwich action. In this case, the distance between AB and DB is less than 200 nm, and AB receives singlet oxygen generated by DB and a light signal with 615 nm is excited. When there is no molecule to be tested, the distance between AB and DB cannot excite the light signal of AB. Therefore, using this principle, LICA can avoid the repeated washing required in traditional ELISA. However, the current LICA technology lacks the capability of multiplex detection. Perkin Elmer company had developed an AlphaPlexTM system [Publication No./Authorized Announcement No.:US8486719], which uses the AB doped with lanthanide complex having different emission wavelengths (615 nm, 545 nm, 645 nm) to detect different molecules to be tested, thus realizing multiplex detection. However, limited kinds of lanthanide complex can be doped into AB, therefore, only several different molecules to be detected can be detected by a single reaction, thus restricting its ability for multiplex detection. In addition, the luminous efficiency of different lanthanide complexes varies greatly, resulting in deviations in the detection results of different molecules to be tested. In US Patent [Application No.: US2013/0210003], DB was doped with photosensitizers having different excitation wavelengths. Different molecules to be tested were detected by different excitation light with different wavelengths. Similar to AlphaPlexTM, the types of photosensitizers with different excitation wavelengths that can be doped into DB are also very limited, which also restricts its multiplex detection capability. Simon et al. reported an aqueous two-phase system and employed LICA technology for multiplex detection. The position of droplets at the bottom of the 96-well plates is regarded as encoding information, and AB and DB for detecting different molecules to be tested were loaded in different droplets to realize multiplex detection. However, the number of droplets in this method, i.e., the number of position codes is limited by the area of the bottom of the plate, so the ability of multiplex detection is also restricted. Moreover, the preparation method of droplets is complicated, and the diffusion rate of the molecules to be tested between aqueous two-phase is relatively low, which further restricts the practical application of the method.

Suspension array technology is most likely to become the mainstream technology of clinical multiple index detection in the future, due to its sensitive and accurate optical encoding identification, fast reaction kinetics, flexible combination of detection items, quantitative analysis, and the like. Encoding microbead is the core of suspension array technology, using microbeads with unique encoding characteristics as reaction units to identify specific target molecules. The fluorescent encoded microbead is the most mainstream among the encoded microbeads. Generally, the inner of the functional microbead is labeled by different fluorescent elements, and the outer surface of the functional microbead is connected to different probe molecules. Then the functional microbead can bind to corresponding different biological targets specifically, and flow cytometry or fluorescence imaging is used as a characterization method to achieve multi-index detection. Luminex company has prepared 100 kinds of fluorescent encoded microbeads by embedding two kinds of organic dyes with different emission wavelengths and different concentration gradients in the 5.6µm polystyrene microbead, which first realize commercialization. Since then, the use of quantum dots as fluorescent encoding elements and the fluorescent encoded microbead based on quantum dot-organic dye hybrid structure have been widely reported. At present, suspension array technology based on the encoded microbead has been widely used in the multi-index detection of nucleic acids, proteins, and other biomolecules. However, the typical suspension array detection process involves tedious multi-step washing and sampling, which brings inconvenience to the detection. Therefore, a washing-free, fast and easy-to-use detection product has a good application prospect for clinical diagnosis.

The embodiments of the present invention will be described below through specific examples. Those skilled in the art can easily understand other advantages and effects of the present invention according to the contents disclosed by the specification. The present invention may also be implemented or applied through other different specific implementation modes. Various modifications or changes may be made to all details in the specification based on different points of view and applications without departing from the spirit of the present invention.

In addition, it should be understood that one or more method steps mentioned in the present invention are not exclusive of other method steps that may exist before or after the combined steps or that other method steps may be inserted between these explicitly mentioned steps, unless otherwise stated; it should also be understood that the combined connection relationship between one or more equipments/devices mentioned in the present invention does not exclude that there may be other equipments/devices before or after the combined equipments/devices or that other equipments/devices may be inserted between these explicitly mentioned equipments/devices, unless otherwise stated. Moreover, unless otherwise stated, the numbering of each method step is only a convenient tool for identifying each method step, and is not intended to limit the order of each method step or to limit the scope of the present invention. The change or adjustment of the relative relationship shall also be regarded as the scope in which the present invention may be implemented without substantially changing the technical content.

Before further describing the specific embodiments of the present invention, it should be understood that the scope of protection of the present invention is not limited to the following specific embodiments; it should also be understood that the terms used in the embodiments of the present invention are just for describing the specific embodiments instead of limiting the scope of the present invention. In the specification and claims of the present invention, the singular forms "a", "one" and "this" also include the plural forms, unless otherwise stated clearly.

When the numerical values are given by the embodiments, it is to be understood that the two endpoints of each numerical range and any one between the two may be selected unless otherwise stated. Unless otherwise defined, all technical and scientific terms used in the present invention have the same meaning as commonly understood by one skill in the art. In addition to the specific method, equipment, and material used in the embodiments, any method, equipment, and material in the existing technology similar or equivalent to the method, equipment, and material mentioned in the embodiments of the present invention may be used to realize the invention according to the grasp of the existing technology and the record of the invention by those skilled in the art.

Unless otherwise stated, the experimental methods, detection methods, and preparation methods disclosed in the present invention all employ conventional techniques of molecular biology, biochemistry, chromatin structure and analysis, analytical chemistry, cell culture, recombinant DNA technology in the technical field, and related fields.

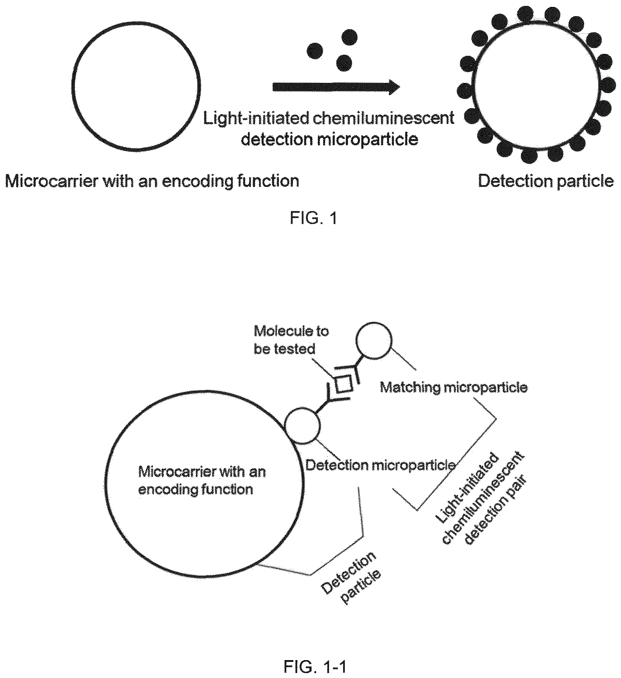

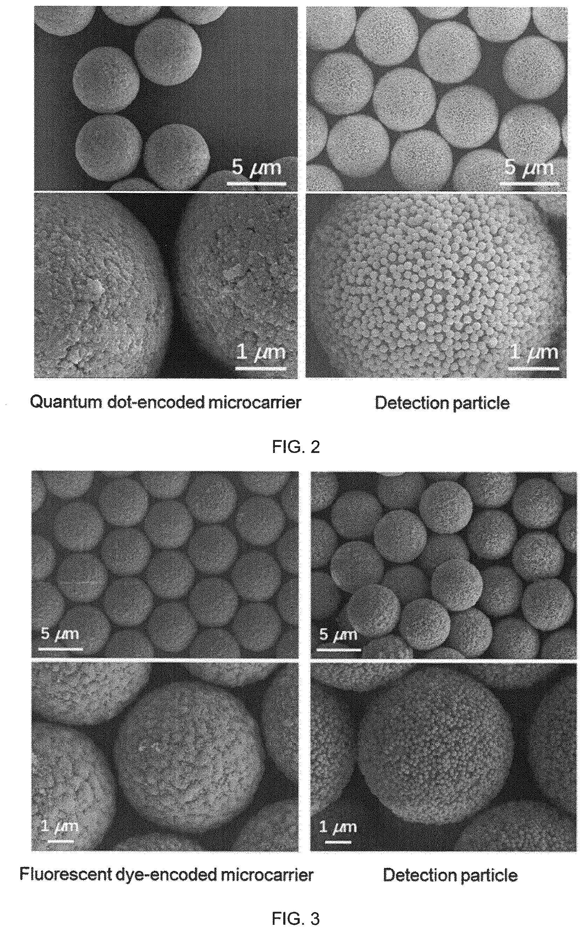

A detection particle suitable for multiplex detection of biomolecules is shown in FIG. 1 and FIG. 2, which includes a microcarrier with an encoding function, a detection microparticle connected to the microcarrier, where the detection microparticle is suitable for light-initiated chemiluminescent detection.

The microcarrier with the encoding function may be selected from one or more of a fluorescent encoded microcarrier, a Raman signal encoded microcarrier, a photonic crystal encoded microcarrier, and a pattern encoded m icrocarrier.

The microcarrier may be a microbead or other-shaped carrier.

The microcarrier is a microbead, and the surface of the microbead is modified with polyethyleneimine (PEI) to provide a reaction site for the subsequent connection of acceptor microparticles.

In one embodiment, the molecular weight of PEI is 10K-750K.

In one embodiment, the molecular weight of PEI is 10K-50K.

In one embodiment, the molecular weight of PEI is 10K.

In one embodiment, the molecular weight of PEI is 30K.

In one embodiment, the molecular weight of PEI is 50K-300K.

In one embodiment, the molecular weight of PEI is 150K.

In one embodiment, the molecular weight of PEI is 300K-750K.

Different molecules to be tested can be identified by the code of the microcarrier with the encoding function.

The microcarrier with the encoding function is selected from magnetic microcarrier and non-magnetic microcarrier.

The microcarrier with the encoding function may have magnetism. It is beneficial to the separation and purification of microcarriers in the detection process. Meanwhile, the preparation process can be simplified, and automatic preparation and automatic detection of detection particles in the future can be facilitated.

The microcarrier with the encoding function may be covered by a polyelectrolyte shell layer.

In one embodiment, the polyelectrolyte shell layer is self-assembled from layers of a polycationic electrolyte and a polyanionic electrolyte.

In one embodiment, the polycationic electrolyte is PDDA (poly (diallyldimethylammonium chloride)), and the polyanionic electrolyte is PSS (sodium polystyrene sulfonate).

In one embodiment, the polyelectrolyte shell layer has at least two layers.

In one embodiment, the polyelectrolyte shell layer has at least four layers.

The detection microparticle is donor microparticle (DB) or acceptor microparticle (AB) suitable for light-initiated chemiluminescent detection. The acceptor microparticle can emit fluorescence, and the donor microparticle is used to excite the acceptor microparticle to emit light. DB is doped with a photosensitizer, which can produce singlet oxygen after being excited by light. AB is doped with a chemiluminescent agent and a fluorescent agent, where the chemiluminescent agent converts the energy of singlet oxygen into emission light with 360nm, and excites the fluorescent agent to produce fluorescence. The chemiluminescent agent is selected from one or more of dioxane and thioxene.

After being irradiated by laser with 680nm, DB can activate the oxygen in the surrounding environment and convert them into singlet oxygen whose survival time only lasts 4 microseconds. The short survival time determines that the spreading diameter of ionic oxygen is very small (about 200 nm). When there is a molecule to be tested, it forms an immune complex with AB of a conjugated capture antibody and DB of a conjugated detection antibody through a double-antibody sandwich action. In this case, the distance between AB and DB is less than 200 nm, and AB receives the singlet oxygen generated by DB to excite a light signal. For example, the light signal can be 615nm or that of other rare earth or fluorescent molecules, as long as it can be distinguished from the signal of the encoded carrier. When there is no molecule to be tested, the distance between AB and DB is too far to excite the light signal of AB.

In a preferred embodiment, the photosensitizer may be macrocycles such as phthalocyanine or porphyrin.

In an embodiment, the fluorescent agent may be selected from a lanthanide complex. For example, the lanthanide may be one or more of europium, terbium, and samarium.

In one embodiment, the fluorescent agent may be a dye, such as 9,10-bis(phenylethynyl)anthracene or rubrene.

The microcarrier and the detection microparticle may be connected through a linker or may be covalently connected.

The linker may be a polymer capable of coordinating with the detection microparticle.

In one embodiment, the linker is selected from polymers whose functional group includes an amino group or a carboxyl group. The linker may be, for example, polyethyleneimine, polyacrylic acid, polymethacrylic acid, polyacrylamide, etc.

In addition, the detection particle suitable for multiplex detection of biomolecules further includes a biocapture substance disposed on the detection microparticle.

The biocapture substance refers to a substance that can specifically bind to the biomolecule to be tested.

Further, the biocapture substance may be one or more of a biocapture probe, an antigen, an antibody, Protin A, ProtinG, and streptoavidin, for detecting the molecule to be tested. The molecule to be tested may be a protein, a small molecular antigen, or a nucleic acid. The biocapture substance may be a molecular probe that binds specifically to a nucleic acid molecule to be tested.

The microcarrier with the encoding function may be magnetic microcarrier or non-magnetic microcarrier.

As shown in FIG. 1-1 a biomolecule detection system for simultaneous detection of multiple biomolecules at least includes a plurality of the aforementioned detection particles suitable for multiplex detection of biomolecules, and matching microparticles, where the matching microparticles refer to light-initiated chemiluminescent microparticles matched with the detection microparticles in the detection particles.

Specifically, when the detection microparticle of the detection particle suitable for multiplex detection of biomolecules is donor microparticle, the matching particle is acceptor microparticle; and when the detection microparticle of the detection particle suitable for multiplex detection of biomolecules is acceptor microparticle, the matching microparticle is donor microparticle.

The matching particle may be modified by streptavidin.

Further, different biomolecules correspond to different encoding luminescent substance pairs. The encoding luminescent substance pair refers to a combination of an encoding substance of the microcarrier with an encoding function and a light-initiated chemiluminescent substance corresponding to the detection microparticle connected to the microcarrier.

Differences between biomolecules to be tested can be distinguished by detecting the encoding luminescent substance pair.

As shown in FIG. 1-1, when the detection microparticle and the matching microparticle can form a complex with the same molecule to be tested, the detection microparticle and the matching microparticle form a light-initiated chemiluminescent detection pair, and the light-initiated chemiluminescent detection pair is a light-initiated chemiluminescent detection pair corresponding to the detection microparticle.

In the biomolecule multiplex detection system, the light-initiated chemiluminescent substance corresponding to the detection microparticle connected to the microcarrier refers to the doped photosensitizer capable of generating singlet oxygen after light excitation and/or the doped fluorescent agent capable of generating fluorescence after light excitation in the light-initiated chemiluminescent detection pair corresponding to the detection microparticle connected to the microcarrier.

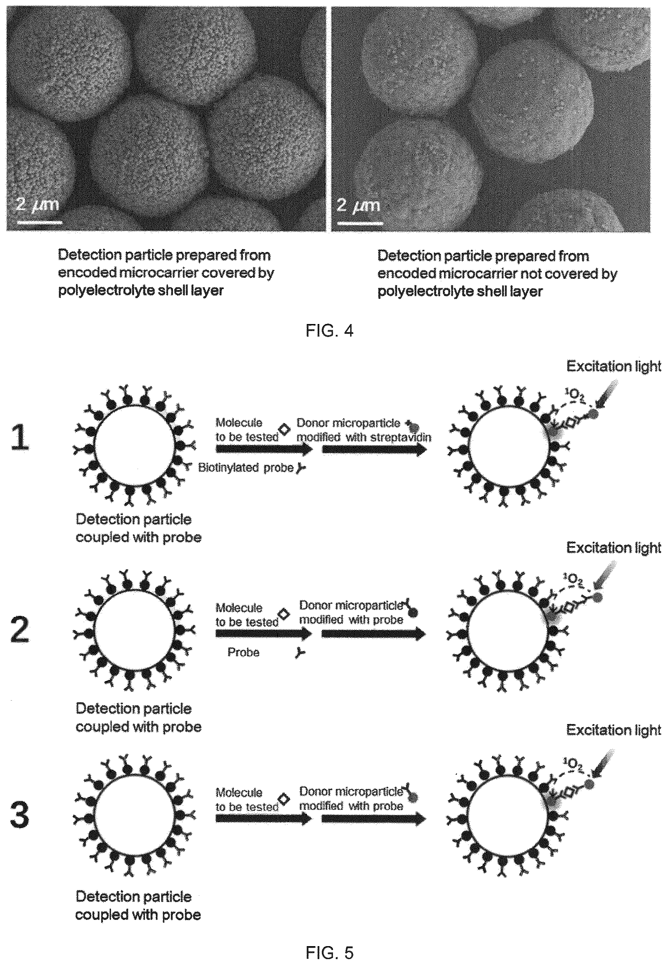

Specifically, as shown in FIG. 5, the matching microparticle is coupled with the biomolecule to be tested when performing a detection, and the molecule to be tested binds to the biocapture substance on the detection particle to form a complex. The formed complex includes two parts, one is the microcarrier with the encoding function, and the other is the light-initiated chemiluminescent detection pair that binds to the molecules to be tested. Different biomolecules to be tested correspond to at least one difference in the code of the microcarrier with the encoding function and the luminescent signal of the light-initiated chemiluminescent detection microparticle pair, so that the biomolecules to be tested can be distinguished. The luminescent signal includes wavelength and/or intensity. Further, the encoding luminescent signal of the microcarrier with the encoding function includes wavelength and/or intensity, and the luminescent signal of the light-initiated chemiluminescent detection microparticle pair is a wavelength, where the luminescent signal of the light-initiated chemiluminescent detection acceptor microparticle is emission wavelength, and the luminescent signal of the light-initiated chemiluminescent detection donor microparticle is excitation wavelength.

In one embodiment, in the biomolecule multiplex detection system, the light-initiated chemiluminescent substance includes two or more light-initiated chemiluminescent substances with different emission wavelengths and/or excitation wavelengths.

In addition, the system further includes a fluorescence microscope with a camera or photographic function. The fluorescence microscope is equipped with at least two excitation light sources, one for excitation of the signal of the microcarrier with the encoding function, and the other for excitation of the light-initiated chemiluminescent signal.

The signal of the microcarrier with the encoding function and the light-initiated chemiluminescent signal are collected by the camera after passing through a filter set. An ordinary fluorescence mode or a time-resolved fluorescence mode may be used to excite the signal of the detection microparticle. In the ordinary fluorescence mode, an excitation light irradiation and a camera exposure are synchronized. In the time-resolved fluorescence mode, the light source is turned off after the excitation light irradiates for a period of time, and then the camera starts to expose and collect signals. This can further reduce the background noise generated by the excitation light.

The excitation light source may be a laser, a xenon lamp, a mercury lamp, or a light-emitting diode (LED).

The camera or photographic function can be realized by a camera. For example, the camera or photographic function may be realized by a charge-coupled device (CCD), an electron-multiplying charge-coupled device (EMCCD), a complementary metal-oxide-semiconductor image sensor (CMOS), or a scientific complementary metal-oxide-semiconductor image sensor (sCMOS).

The signal of the microcarrier with the encoding function may be a fluorescent signal, and the signal of the light-initiated chemiluminescent substance may be a light-initiated chemiluminescent signal.

The system further includes an image processing device, which is used to match the image obtained after excitation of the signal of the microcarrier with the encoding function with the image obtained after excitation of the light-initiated chemiluminescent signal. The positions of the fluorescent signal and the light-initiated chemiluminescent signal in the image can be unified, so that the position of the microcarrier corresponds to that of the light-initiated chemiluminescent detection microparticle pair connected to the microcarrier in the obtained composite, meanwhile, the emitted signals can be collected.

Image processing can be performed by the following steps: ① image pre-processing: performing noise reduction on the resulted fluorescent image and the light-initiated chemiluminescent image, distinguishing the fluorescent signal or the light-initiated chemiluminescent signal from the background signal, and identifying the positions of the signals in the image by global thresholding. ② co-localization: unifying the positions of the fluorescent signal and the light-initiated chemiluminescent signal in the image. ③ gray analysis: reading the gray value of the fluorescent signal and the light-initiated chemiluminescent signal respectively. ④\ cluster analysis: clustering the fluorescent signals at different positions in the image according to the intensity of gray value, and obtaining different classifications, which are used as the categories of different molecules to be tested.

The present invention provides a method for preparing the detection particle suitable for multiplex detection of biomolecules, including the following operations:
Mixing the microcarrier with the encoding function and a dispersion containing the detection microparticle, performing a reaction protected from light, and obtaining the detection particle suitable for multiplex detection of biomolecules.

In one embodiment, the microcarrier with the encoding function can be added dropwise into the dispersion containing the detection microparticle.

The method further includes the following step: the microcarrier with the encoding function is added with linkers before being added dropwise into the dispersion containing the detection microparticle.

The method further includes the following step: coupling the biocapture substance on the surface of the detection microparticle. The biocapture substance may be one or more of a biocapture probe, an antigen, an antibody, Protin A, ProtinG, and streptoavidin. The biocapture probe may be a molecule probe that binds specifically to a nucleic acid molecule to be tested.

In the dispersion containing the detection microparticle, the dispersant is MES (2-(N-morpholino) ethanesulfonic acid buffer) or MEST solution (2-(N-morpholino) ethanesulfonic acid buffer containing 0.1% Tween).

The aforementioned detection particle suitable for multiplex detection of biomolecules or the aforementioned biomolecule detection system can be used in biomolecule detection.

As shown in FIG. 5, the biomolecule detection method of the present invention includes the following steps:
(1) mixing the detection particle suitable for multiplex detection of biomolecules with a sample solution to be tested, and incubating;
(2) adding the light-initiated chemiluminescent detection microparticle matched with the detection microparticle, and incubating;
(3) placing the mixture obtained in step (2) in the fluorescence microscope with a camera or photographic function to perform detection, and exciting the signal of the microcarrier with the encoding function and the signal of the detection microparticle (i.e., light-initiated chemiluminescent signal);
(4) collecting the signal of the microcarrier with the encoding function and the light-initiated chemiluminescent signal, respectively, and matching the image obtained after excitation of the signal of the microcarrier with the encoding function with the image obtained after excitation of the light-initiated chemiluminescent signal.

In addition, the detection particle suitable for multiplex detection of biomolecules further includes the biocapture substance.

In an embodiment, the biocapture substance may also be added in step (1).

The excitation of the signal of the detection microparticle can be achieved by an ordinary fluorescence mode or a time-resolved fluorescence mode. In the ordinary fluorescence mode, an excitation light irradiation and a camera exposure are synchronized. In the time-resolved fluorescence mode, the light source is turned off after the excitation light irradiates for a period of time, and then the camera starts to expose and collect signals. The time-resolved fluorescence mode can further reduce the background noise generated by the excitation light.

The positions of the fluorescent signal and the light-initiated chemiluminescent signal in the image are unified, so that the position of the microcarrier corresponds to that of the light-initiated chemiluminescent detection microparticle pair connected to the microcarrier in the obtained composite, meanwhile, the signals can be collected.

Image processing can be performed by the following steps: ① image pre-processing: performing noise reduction on the resulted fluorescent image and the light-initiated chemiluminescent image, distinguishing the fluorescent signal or the light-initiated chemiluminescent signal from the background signal, and identifying the positions of the signals in the image by global thresholding. ② co-localization: unifying the positions of the fluorescent signal and the light-initiated chemiluminescent signal in the image. ③ gray analysis: reading the gray value of the fluorescent signal and the light-initiated chemiluminescent signal, respectively. ④ cluster analysis: clustering the fluorescent signals at different positions in the image according to the intensity of gray value, and obtaining different classifications, which are used as the categories of different molecules to be tested.

In step (4), the signal of the microcarrier with the encoding function and the light-initiated chemiluminescent signal are collected by the camera after passing through a filter set.

In step (1) or (2), washing or washing-free may be adopted after incubating.

In step (4), the signal of the microcarrier with the encoding function and the light-initiated chemiluminescent signal are collected by the camera after passing through a filter set.

The microcarrier is selected from magnetic microcarrier and non-magnetic microcarrier.

In one embodiment, in step (1), when the microcarrier is magnetic microcarrier, incubating after mixing, and performing a magnetic separation and a washing.

In one embodiment, in step (1) or (2), when the microcarrier is magnetic microcarrier, performing a magnetic separation and a washing after incubating.

As shown in FIG. 3, in step (2), the matching microparticle is modified with the biocapture substance.

In one embodiment, the biomolecule detection method includes performing a signal detection after mixing the detection particle suitable for multiplex detection of biomolecules coupled with the specific biocapture substance, the biotinylated biocapture substance, the molecule to be tested, and the matching microparticle modified with streptavidin.

In one embodiment, the biomolecule detection method includes performing a signal detection after mixing the detection particle suitable for multiplex detection of biomolecules coupled with the biocapture substance, the biocapture substance, the molecule to be tested, and the matching microparticle modified with the biocapture substance.

In one embodiment, the biomolecule detection method includes performing a signal detection after mixing the detection particle suitable for multiplex detection of biomolecules coupled with the biocapture substance, the molecule to be tested, and the matching microparticle modified with the biocapture substance.

For the molecules to be tested with low abundance, performing a multi-step washing to further improves the detection sensitivity. Therefore, the introduction of multi-step washing can also alleviate the "hook" effect, thus expanding the dynamic range of detection.

The method described in the present invention may be used for non-diagnostic or non-therapeutic purposes.

### Embodiment 1 Assembly Of Encoded Microcarriers With Quantum Dots On The Surface And Acceptor Microparticles With Carboxyl On The Surface

Step 1: 1.25mg of the encoded microcarrier with two-color quantum dots on the surface was subjected to magnetic separation to remove the supernatant, washed with 300µL MEST solution for three times, and then dispersed in 125µL MEST solution to obtain a microcarrier dispersion. 0.6mg of acceptor microparticle were centrifuged to remove the supernatant and then ultrasonically dispersed in 125µL MEST solution to obtain an acceptor microparticle dispersion.

Step 2: The microcarrier dispersion obtained in step 1 is added dropwise into the acceptor microparticle dispersion under the ultrasonic condition until the mixture is uniform, and then the mixture is rotated and mixed for 0.5h. 5mg carbodiimide (EDC) and 5mg N-hydroxysuccinimide (NHS) were added and dissolved in 100µL MEST solution, then EDC/NHS solution was added into the mixed solution of microcarrier dispersion and acceptor microparticle dispersion, mixed through vortex until the distribution is even, and rotated and reacted for 3 hours in a dark room. After the reaction, the supernatant was removed by magnetic separation, the left part was washed for three times with 2.5mM sodium hydroxide (NaOH), washed for three times with ultrapure water, and dispersed in 300µL ultrapure water to obtain the detection particle with the encoding function suitable for multiplex detection of biomolecules.

When microcarriers with different diameters are used, the assembly method of microcarrier and acceptor microparticle is basically the same as the above method, and only the amount of acceptor microparticle and the amount of related reactants need to be slightly adjusted according to the change of the surface area of the microcarrier.

The acceptor microparticle is nanosphere internally doped with thioxene and lanthanide complex, which is purchased from PerkinElmer Company. The acceptor microparticle has a particle size of 200nm, and a surface group of carboxyl or aldehyde. As shown in FIG. 1 and 2, the amino group on the surface of the microcarrier with the encoding function (see the method mentioned in [*Chem.Mater*.2017,29,10398.] for preparation, where the molecular weight of PEI was 10K, the encoded microcarrier with two-color quantum dots on the surface was covered by a polyelectrolyte shell layer, and the polyelectrolyte shell layer was self-assembled by layers of PDDA (poly dimethyl diallyl ammonium chloride) and PSS (poly(sodium 4-styrenesulfonate))) is covalently coupled to the carboxyl or aldehyde on the surface of the acceptor microparticle to obtain the detection particle suitable for multiplex detection of biomolecules.

### Embodiment 2 Assembly Of Encoded Microcarriers Internally Filled With Fluorescent Dye (The Surface Of The Encoded Microcarriers Being Covered With The Polyelectrolyte Shell Layer) And Acceptor Microparticles With Carboxyl On The Surface

Step 1: Mesoporous polystyrene microbeads with a diameter of 5.5µm were used as microcarriers, combinations of two fluorescent dyes (fluorescein isothiocyanate (FITC) and rhodamine isothiocyanate (RITC)) with different concentration were prepared, and magnetic response characteristic was imparted to the microbeads through self-assembly of magnetic nanoparticles, then 12 kinds of encoded microcarriers internally filled with fluorescent dyes were obtained (see the method mentioned in [Publication No.:CN 112111268A] for preparation).

Step 2: The encoded microcarriers were covered with polyelectrolyte on the surface. 3mg of the encoded microcarriers covered by silica on the surface in step 1 were added into a 2mL centrifuge tube, washed with pure water, added with 1mL of 5mg/mL PDDA (poly dimethyl diallyl ammonium chloride) NaCl solution, reacted for 0.25h rotatedly in the dark, washed once with ultrapure water after the reaction, then added with 1mL of 5mg/mL PSS (poly(sodium 4-styrenesulfonate)) NaCl solution, reacted for 0.25h rotatedly in the dark, and washed once with ultrapure water after the reaction. The above steps were repeated twice, so that a total of at least 4 layers of polyelectrolyte are alternately coated on the surfaces of the microbeads. Then 1mL of 50mg/mL PEI solution was added, the mixture was dispersed through a vortex and reacted for 0.5h in a dark room, so that the surfaces of microbeads were modified with a layer of PEI to provide reaction sites for subsequent connection of acceptor microparticles. After the reaction, the supernatant was removed by magnetic separation, the left part was washed for 3 times with ultrapure water, dispersed in 300µL of ultrapure water, and stored in a cool place to obtain encoded microcarriers covered by polyelectrolyte. The molecular weight of PEI was 30K. The concentration of NaCl solution was 0.1M-1.0M.

Step 3: 1.25mg of the two-color encoded microcarrier in step 2 was subjected to magnetic separation to remove the supernatant, the left part was washed with 300µL MEST solution for three times, and then dispersed in 125µL MEST solution to obtain an encoded microcarrier dispersion. 0.6mg of acceptor microparticles was centrifuged to remove the supernatant and then the left part was ultrasonically dispersed in 125µL MEST solution to obtain an acceptor microparticle dispersion.

Step 4: The microcarrier dispersion obtained in step 3 was added dropwise to the acceptor microparticle dispersion under ultrasonic conditions until the mixture is uniform, then rotated and mixed for 0.5h. 5mg carbodiimide (EDC) and 5mg N-hydroxysuccinimide (NHS) were added and dissolved in 100µL MEST solution, then the EDC/NHS solution was added into the mixed solution of the microcarrier dispersion and the acceptor microparticle dispersion, mixed through a vortex until the distribution is even, and rotated and reacted for 3 hours in a dark room. After the reaction, the supernatant was removed by magnetic separation, the left part was washed for three times with 2.5mM sodium hydroxide (NaOH), washed for three times with ultrapure water, and dispersed in 300 µ L ultrapure water to obtain the detection particle with the encoding function suitable for multiplex detection of biomolecules.

When microcarriers with different diameters are used, the assembly method of microcarrier and acceptor microparticle is basically the same as the above method, and only the amount of acceptor microparticle and the amount of related reactants need to be slightly adjusted according to the change of the surface area of the microcarrier.

FIG. 3 is a schematic diagram of scanning electron microscopic images for the microcarriers internally filled with fluorescent dye and the detection particle without biocapture substances.

### Embodiment 3 Assembly Of Encoded Microcarriers Internally Filled With Fluorescent Dye (The Surface Of The Encoded Microcarriers Being Not Covered By Polyelectrolyte Shell Layer) And Acceptor Microparticles With Carboxyl On The Surface

Step 1: Mesoporous polystyrene microbeads with a diameter of 5.5µm were used as microcarriers, combinations of two fluorescent dyes (FITC and RITC) with different concentration were prepared, and magnetic response characteristic was imparted to the microbeads through self-assembly of magnetic nanoparticles, then 12 kinds of encoded microcarriers internally filled with fluorescent dyes were obtained (see the method mentioned in [Publication No.:CN 112111268A] for preparation).

Step2: 3mg of the encoded microcarriers with silica coated on the surface in step1 was added in a 2mL centrifuge tube, washed with pure water, added with 1mL of 50mg/mL PEI solution, dispersed through a vortex, and reacted for 0.5h in a dark room, so that the surfaces of microbeads were modified with a layer of PEI to provide reaction sites for subsequent connection of acceptor microparticles. After the reaction, the supernatant was removed by magnetic separation, and the left part was washed for 3 times with ultrapure water, and dispersed in 300µL ultrapure water. The molecular weight of PEI was 150K. The concentration of NaCl solution was 0.1M-1.0M. 1.25mg of the obtained two-color encoded microcarrier was subjected to magnetic separation to remove the supernatant, the left part was washed with 300µL MEST solution for three times, and then dispersed in 125µL MEST solution to obtain an encoded microcarrier dispersion. 0.6mg of acceptor microparticles were centrifuged to remove the supernatant and then the left part was ultrasonically dispersed in 125µL MEST solution to obtain an acceptor microparticle dispersion.

Step 3: The microcarrier dispersion obtained in step 2 is added dropwise into the acceptor microparticle dispersion under the ultrasonic conditions until the mixture is uniform, and then the mixture is rotated and mixed for 0.5h. 5mg carbodiimide (EDC) and 5mg N-hydroxysuccinimide (NHS) were added and dissolved in 100µL MEST solution, then the EDC/NHS solution was added into the mixed solution of the microcarrier dispersion and the acceptor microparticle dispersion, mixed through a vortex until the distribution is even, and rotated and reacted for 3 hours in a dark room. After the reaction, the supernatant was removed by magnetic separation, the left part was washed for three times with 2.5mM sodium hydroxide (NaOH), washed for three times with ultrapure water, and dispersed in 300µL ultrapure water to obtain the detection particle with the encoding function suitable for multiplex detection of biomolecules.

When microcarriers with different diameters are used, the assembly method of microcarrier and acceptor microparticle is basically the same as the above method, and only the amount of acceptor microparticle and the amount of related reactants need to be slightly adjusted according to the change of the surface area of the microcarrier.

FIG. 4 shows a comparison of scanning electron microscopic images of the detection particles prepared from the encoded microcarrier covered by multiple polyelectrolyte shell layers and the detection particles prepared from the encoded microcarrier not covered by a polyelectrolyte shell layer. It can be seen that the surface of the detection particles prepared from the encoded microcarrier covered by multiple polyelectrolyte shell layers is covered with more acceptor microparticles. Since the number of acceptor microparticles loaded on the encoded microcarriers is directly related to the intensity of the detection signal, the multilayer polyelectrolyte can improve the sensitivity and dynamic range of the detection.

### Embodiment 4 Assembly Of Encoded Microcarriers And Acceptor Microparticles With Aldehyde On The Surface

Step 1: 1.25mg of the encoded microcarrier with quantum dots on the surface or internally filled with fluorescent dye was subjected to magnetic separation to remove the supernatant, the left part was washed with 300µL MEST solution for three times, and then dispersed in 125µL MEST solution to obtain a microcarrier dispersion. 0.6mg of acceptor microparticle was centrifuged to remove the supernatant, and the left part was ultrasonically dispersed in 125µL MEST solution to obtain an acceptor microparticle dispersion, then 15µL NaCNBH3 (25 mg/ml) was added. The encoded microcarrier with quantum dots on the surface can be prepared by referring to [Chem. Mater.2017,29,10398.]*,* where the encoded microcarrier with quantum dots on the surface was covered by a polyelectrolyte shell layer, and the polyelectrolyte shell layer was formed from self-assembly of layers of PDDA (poly dimethyl diallyl ammonium chloride) and PSS (poly(sodium 4-styrenesulfonate)). The encoded microcarrier internally filled with fluorescent dye can be prepared by referring to the patent No.112111268A.

Step 2: The microcarrier dispersion obtained in step 1 was added dropwise into the acceptor microparticle dispersion obtained in step 1 under the ultrasonic conditions. After addition, the mixture was oscillated through a vortex and placed in a thermostat at 37°C, and rotated for 48 hours in a rotating mixer protected from light. After the reaction, the supernatant was removed by magnetic separation, the left part was washed for three times with 2.5mM sodium hydroxide (NaOH), washed for three times with ultrapure water, and dispersed in 300µL ultrapure water to obtain the detection particle with the encoding function suitable for multiplex detection of biomolecules.

When microcarriers with different diameters are used, the assembly method of microcarrier and acceptor microparticle is basically the same as the above method, and only the amount of acceptor microparticle and the amount of related reactants need to be slightly adjusted according to the change of the surface area of the microcarrier.

### Embodiment 5 Biological Coupling Of The Detection Particle Prepared From Acceptor Microparticle With Carboxyl On The Surface

Step 1: 0.75mg of the detection particles prepared in Embodiment 1 or 2 were added into two centrifuge tubes labeled "0" and "1" respectively, where "0" is the control group, and "1" is the experimental group. Each tube was added with 400µL of MEST solution (10mM, pH 5.0), and then washed for three times with 400µL of MEST solution;

Step 2: Carboxyl activation: Each tube was added with 200µL of the prepared NHS (50 mg/mL) and 200µL of EDC (50 mg/mL), then each tube was mixed until the distribution was uniform. The two groups of tubes were placed on a rotating mixer for rotating for 20 min (room temperature). The detection particles were adsorbed by the magnetic stand, the supernatant was removed, and the left part was washed twice with 400µL MEST solution.

Step 3: 400µL of MEST solution (10mM, pH 5.0) was added to the "0" tube, 400µL of capture antibody solution (0.5 mg/mL) was added to the "1" tube, the two groups of tubes were placed in a thermostat at 37°C and rotated for 2 hours in a rotating mixer.

Step 4: The detection particles were adsorbed by the magnetic stand, and the supernatant was retained for a quantitative test of BCA protein. The detection particles were washed for 3 times with 400µL blocking solution (PBS buffer containing 0.3% glycine and 0.5% BSA), and then 400µL of blocking solution was added to block the detection particles overnight in a refrigerator at 4°C.

Step 5: After blocking, the detection particles were adsorbed by the magnetic stand, and the supernatant was removed by pipette, the left part was washed with 400µL preservation solution (PBS buffer containing 0.1% BSA) for three times, 50µL of preservation solution was added to each tube for storage, and a flow cytometer was used for counting.

7 kinds of detection particles with different codes were selected. According to the above biological coupling method, the capture antibodies of interferon-γ (IFN-γ), interleukin-2 (IL-2 ), tumor necrosis factor-α (TNF-α), interleukin-4 (IL-4), interleukin-9 (IL-9), interleukin-10(IL-10), and interleukin-17A (IL-17A) werecoupled on the surface of the detection particles respectively to obtain detection particles modified with different capture antibodies.

### Embodiment 6 Biological Coupling Of The Detection Particle Prepared From Acceptor Microparticle With Aldehyde On The Surface

Step 1: 0.75 mg of the detection particles prepared in Embodiment 4 were respectively added into two centrifuge tubes labeled "0" and "1", where "0" is the control group, and "1" is the experimental group. Each tube was added with 400µL of MEST solution (10mM, pH 5.0), and then washed for three times with 400µL of MEST solution;

Step 2: Each tube was added with 15µL. of the prepared NaCNBH3 (25 mg/ml), then each tube was mixed well. 400µL of MEST solution was added to the "0" tube, 400µL of capture antibody solution (0.5 mg/mL) was added to the "1" tube, the two groups of tubes were placed in a thermostat at 37°C and rotated for 48 hours in a rotating mixer.

Step 3: The detection particles were adsorbed by the magnetic stand, and then the supernatant was retained for a quantitative test of BCA protein. The detection particles were washed for 3 times with 400µL blocking solution (PBS buffer containing 0.3% glycine and 0.5% BSA), and then 400µL of blocking solution was added to block the detection particles overnight in a refrigerator at 4°C.

Step 4: After blocking, the detection particles were adsorbed by the magnetic stand, and the supernatant was removed by pipette, the left part was washed with 400µL preservation solution (PBS buffer containing 0.1% BSA) for three times, 50µL of preservation solution was added to each tube for storage, and a flow cytometer was used for counting.

7 kinds of detection particles with different codes were selected. According to the above biological coupling method, the capture antibodies of interferon-γ (IFN-γ), interleukin-2 (IL-2), tumor necrosis factor-α (TNF-α), interleukin-4 (IL-4), interleukin-9 (IL--9), interleukin-10(IL-10), and interleukin-17A (IL-17A) were coupled on the surface of the detection particles respectively to obtain detection particles modified with different capture antibodies.

### Embodiment 7 A Standard Curve Of IFN-γ Detection Under A "washing-free" Strategy By Using The Detection Particle Prepared From The Microcarrier Filled With Fluorescent Dye

Step 1. Preparation of a detection particle solution modified with the capture antibody: 2000 detection particles coupled with the capture antibody of interferon-γ (IFN-γ) on the surface in Embodiment 5 were dispersed in 20µL PBS buffer containing 0.5% BSA and 0.05% Tween-20.

Step 2. Preparation of antigen standard solution: IFN-γ antigen standard was diluted to 104, 103, 102, 10, 10-1, 10-2, 10-3, 0 ng/mL.

Step 3. Preparation of biotin-modified detection antibody solution: biotin-modified IFN-γ detection antibody was diluted to a concentration of 5ug/mL.

Step 4. Preparation of a donor microparticle solution modified with streptavidin: the donor microparticle modified with streptavidin was diluted to 1mg/mL.

Step 5. As shown in FIG. 5, the detection particle solution modified with the capture antibody in step 1 was mixed with 40µL of the antigen standard solution in step 2, then 20µL of the biotin-modified detection antibody solution in step 3 was added, and the obtained solution was incubated at 37°C for 30min under rotation. Then, 10µL of the donor microparticle solution modified with streptavidin in step 4 was added, and the obtained solution was incubated at 37°C for 60min under rotation. The obtained solution was placed directly on the instrument for imaging detection, and the encoding signal of the microcarrier and the signal of the detection microparticle of each detection particle were collected and counted respectively.

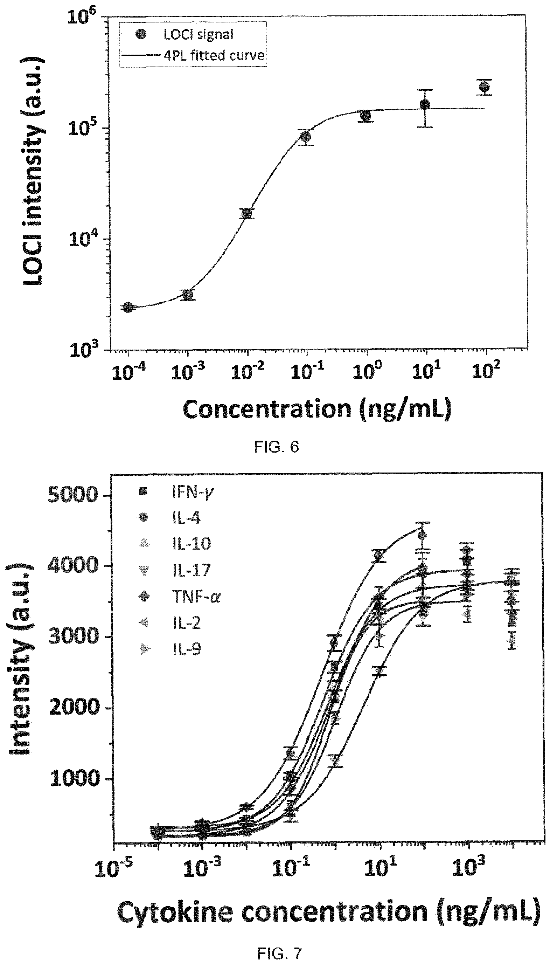

Step 6. As shown in FIG. 6, the standard curve of IFN-γ antigen was drawn with the concentration of IFN-γ antigen as the horizontal coordinate and the average signal intensity of the detection microparticle of the detection particle as the vertical coordinate.

### Embodiment 8 A Standard Curve Of Multiple Protein Detection Under A "washing-free" Strategy By Using The Detection Particle Prepared From The Microcarrier Encoded With Quantum Dots

Since the detection particles in the present invention have a magnetic adsorption property, two strategies of "washing-free" and "washing" can be flexibly selected according to actual needs. The characteristic of "washing-free" strategy is fast and simple, while the characteristic of "washing" strategy is more sensitive, resulting in a delay of the "hook" effect, and a wider dynamic range.

Step 1. Preparation of a detection particle solution modified with the capture antibody: 2000 of each kind of the 7 kinds of detection particles in Embodiment 5 were dispersed in 20µL PBS buffer containing 0.5% BSA and 0.05% Tween-20.

Step 2. Preparation of antigen standard solutions: IFN-γ, IL-2, TNF-α, IL-4, IL-9, IL-10, and IL-17A antigen standards were diluted to 104, 103, 102, 10, 10-1, 10-2, 10-3, 0 ng/mL, and the standards with the same concentration were mixed.

Step 3. Preparation of biotin-modified detection antibody solutions: biotin-modified IFN-γ, IL-2, TNF-α, IL-4, IL-9, IL-10, and IL-17A detection antibodies were diluted to a concentration of 5ug/mL respectively and then were mixed.

Step 4. Preparation of a donor microparticle solution modified with streptavidin: the donor microparticle modified with streptavidin was diluted to 1mg/mL.

Step 5. As shown in FIG. 5, the detection particle solution modified with the capture antibody in step 1 was mixed with 40µL of the antigen standard solution in step 2, and then 20µL of the biotin-modified detection antibody solution in step 3 was added, and the obtained solution was incubated at 37°C for 30 min under rotation. Then, 10µL of the donor microparticle solution modified with streptavidin in step 4 was added, and the obtained solution was incubated at 37°C for 60 min under rotation. The obtained solution was placed directly on the instrument for imaging detection, and the encoding signal of the microcarrier and the signal of the detection microparticle of each detection particle were collected and counted respectively.

Step 6. As shown in FIG. 7, the standard curve of each antigen was drawn with the concentration of antigen as the horizontal coordinate and the average signal intensity of the detection microparticle of the detection particle as the vertical coordinate.

### Embodiment 9 A Standard Curve Of Multiple Protein Detection Under A "washing" Strategy By Using The Detection Particle Prepared From The Microcarrier Encoded With Quantum Dots

Step 1. Preparation of a detection particle solution modified with the capture antibody: 2000 of each kind of the 7 kinds of detection particles in Embodiment 5 were dispersed in 20µL PBS buffer containing 0.5% BSA and 0.05% Tween-20.

Step 2. Preparation of antigen standard solutions: IFN-γ, IL-2, TNF-α, IL-4, IL-9, IL-10, and IL-17A antigen standards were diluted to 104, 103, 102, 10, 10-1, 10-2, 10-3, 0 ng/mL, and the standards with the same concentration were mixed.

Step 3. Preparation of biotin-modified detection antibody solutions: biotin-modified IFN-γ, IL-2, TNF-α, IL-4, IL-9, IL-10, and IL-17A detection antibodies were diluted to a concentration of 5ug/mL respectively and then were mixed.

Step 4. Preparation of a donor microparticle solution modified with streptavidin: the donor microparticle modified with streptavidin was diluted to 1mg/mL.

Step 5. The detection particle solution modified with the capture antibody in step 1 was mixed with 40µL of the antigen standard solution in step 2, and the obtained solution was incubated at 37°C for 30 min under rotation, then washed with PBS buffer containing 0.05% Tween-20 for 3 times. Then 20µL of the biotin-modified detection antibody solution in step 3 and 60µL of PBS buffer containing 0.5% BSA and 0.05% Tween-20 were added, the obtained solution was incubated at 37°C for 30 min under rotation, and washed with PBS buffer containing 0.05% Tween-20 for 3 times by magnetic adsorption. And then 10 µL of donor microparticle solution modified with streptavidin in step 4 and 60pL of PBS buffer containing 0.5% BSA and 0.05% Tween-20 were added, the obtained solution was incubated at 37°C for 60 min under rotation, washed with PBS buffer containing 0.05% Tween-20 for 3 times by magnetic adsorption, afterward resuspended in 100µL of PBS buffer containing 0.5% BSA and 0.05% Tween-20. The obtained solution was placed directly on the instrument for imaging detection, and the encoding signal of the microcarrier and the signal of the detection microparticle of each detection particle were collected and counted respectively.

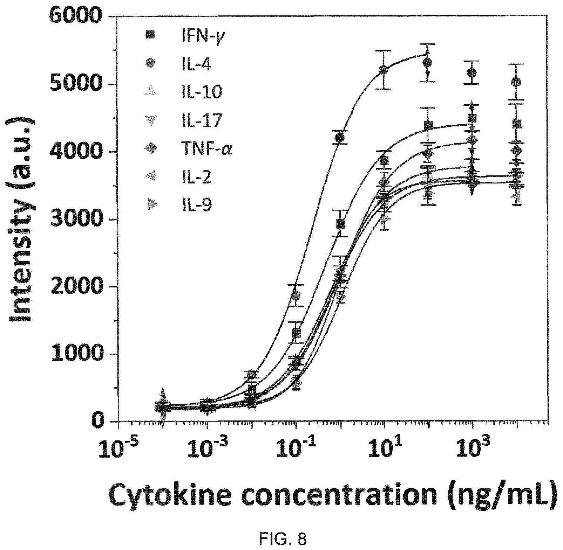

Step 6. As shown in FIG. 8, the standard curve of each antigen was drawn with the concentration of antigen as the horizontal coordinate and the average signal intensity of the detection microparticle of the detection particle as the vertical coordinate.

### Embodiment 10 Clinical Sample Detection: Detecting 7 Antigens Secreted From Leukocytes In Blood Samples By Using A "washing-free" Strategy

Step 1. Preparation of a detection particle solution modified with the capture antibody: 2000 of each kind of the 7 kinds of detection particles in Embodiment 5 were dispersed in 20µL PBS buffer containing 0.5% BSA and 0.05% Tween-20.

Step 2. Sample processing: peripheral blood mononuclear cells were extracted from the patient's whole blood by using density gradient centrifugation and added to the 96-well plates with about 2.5x105 cells in each well, then 1µg/mL of lipopolysaccharide was added to stimulate the cells to secrete antigen , and finally the supernatant was collected after removing the cells by centrifugation.

Step 3. Preparation of biotin-modified detection antibody solutions: biotin-modified IFN-γ, IL-2, TNF-α, IL-4, IL-9, IL-10, and IL-17A detection antibodies were diluted to a concentration of 5ug/mL respectively and then were mixed.

Step 4. Preparation of a donor microparticle solution modified with streptavidin: the donor microparticle modified with streptavidin was diluted to 1mg/mL.

Step 5. The detection particle solution modified with the capture antibody in step 1 was mixed with 40µL of the supernatant in step 2, then 40µL of the biotin-modified detection antibody solution in step 3 was added, and the obtained solution was incubated at 37°C for 30 min under rotation, Then, 10µL of the donor microparticle solution modified with streptavidin in step 4 was added, and the obtained solution was incubated at 37°C for 60 min under rotation. The obtained solution was placed directly on the instrument for imaging detection, and the encoding signal of the microcarrier and the signal of the detection microparticle of each detection particle were collected and counted respectively.

Step 6. As shown in Table 1, the concentration of antigen can be obtained through the corresponding signal of detection microparticle of each kind of detection particle in the standard curve in FIG. 7.

**Table 1**

| sample No. | concentration of samples to be tested (ng/mL) | | | | | | |
|---|---|---|---|---|---|---|---|
| | IFN-γ | IL-4 | IL-10 | IL-17A | TNF-α | IL-2 | IL-9 |
| 1 | 0.66 | 0.71 | 0.08 | 0.1 | 0.46 | 0.11 | 0.2 |
| 2 | 0.38 | 0.61 | 0.09 | 0.63 | 0.23 | 0.17 | 0.98 |
| 3 | 0.41 | 0.35 | 0.12 | 0.54 | 0.37 | 0.13 | 0.39 |
| 4 | 0.35 | 0.63 | 0.15 | 0.09 | 0.11 | 0.09 | 0.13 |
| 5 | 0.49 | 0.59 | 0.11 | 0.42 | 0.18 | 0.15 | 0.52 |
| 6 | 1.63 | 0.65 | 0.81 | 1.28 | 0.92 | 1.12 | 1.77 |
| 7 | 1.15 | 0.52 | 1.32 | 2.32 | 0.71 | 0.91 | 3.08 |
| 8 | 2.08 | 0.61 | 1.08 | 3.05 | 1.74 | 1.52 | 2.75 |
| 9 | 1.59 | 0.92 | 1.22 | 0.79 | 1.31 | 1.36 | 1.12 |
| 10 | 0.92 | 0.81 | 0.92 | 1.99 | 1.68 | 1.28 | 2.51 |
| 11 | 0.36 | 0.42 | 0.21 | 0.15 | 0.32 | 0.32 | 0.36 |
| 12 | 0.58 | 0.35 | 0.35 | 0.33 | 0.77 | 0.27 | 0.49 |
| 13 | 0.61 | 0.61 | 0.17 | 0.39 | 0.3 | 0.31 | 0.59 |
| 14 | 0.15 | 0.09 | 0.16 | 0.17 | 0.09 | 0.17 | 0.21 |
| 15 | 0.29 | 0.53 | 0.08 | 0.35 | 0.15 | 0.12 | 0.18 |
| 16 | 1.03 | 0.52 | 1.95 | 1.12 | 0.92 | 2.33 | 1.61 |
| 17 | 0.55 | 0.51 | 0.77 | 1.45 | 0.34 | 1.33 | 1.79 |
| 18 | 1.68 | 0.88 | 1.15 | 0.6 | 0.91 | 1.63 | 1.04 |
| 19 | 1.29 | 0.26 | 0.81 | 1.33 | 1.71 | 1.06 | 1.52 |
| 20 | 1.31 | 0.64 | 0.62 | 1.91 | 0.67 | 0.73 | 1.51 |
| 21 | 0.15 | 0.09 | 0.2 | 0.12 | 0.09 | 0.27 | 0.19 |
| 22 | 0.24 | 0.57 | 0.15 | 0.24 | 0.13 | 0.17 | 0.41 |
| 23 | 0.21 | 0.15 | 0.11 | 0.53 | 0.11 | 0.23 | 0.35 |
| 24 | 0.35 | 0.21 | 0.08 | 0.31 | 0.22 | 0.09 | 0.51 |
| 25 | 0.17 | 0.66 | 0.26 | 0.17 | 0.29 | 0.19 | 0.28 |
| 26 | 1.39 | 1.27 | 1.02 | 0.8 | 0.98 | 1.58 | 1.16 |
| 27 | 0.84 | 0.27 | 1.36 | 1.02 | 0.42 | 1.09 | 1.97 |
| 28 | 1.78 | 0.5 | 2.39 | 1.94 | 2.32 | 2.77 | 1.43 |
| 29 | 0.94 | 0.47 | 1.82 | 0.63 | 0.52 | 1.61 | 1.12 |
| 30 | 1.01 | 0.71 | 0.77 | 1.01 | 1.81 | 0.93 | 1.15 |

**The** above embodiments are intended to illustrate the disclosed embodiments of the invention and should not be understood as restrictions on the invention. In addition, various modifications of the present invention, as well as variations of the methods and compositions of the present invention, will be apparent to those skilled in the art without departing from the scope of the present invention. While the invention has been described in detail in connection with various specific preferred embodiments thereof, however, it should be understood that the present invention should not be limited to these specific embodiments. In fact, various modifications to the invention as apparent to those skilled in the art are intended to be included within the scope of the invention.

## Claims

1. A detection particle suitable for multiplex detection of biomolecules, comprising
a microcarrier with an encoding function,
detection microparticles connected to the microcarrier, wherein
the detection microparticles are suitable for light-initiated chemiluminescent detection.

2. The detection particle suitable for multiplex detection of biomolecules according to claim 1, further comprising one or more of the following:
a. the microcarrier with the encoding function is selected from one or more of a fluorescent encoded microcarrier, a Raman signal encoded microcarrier, a photonic crystal encoded microcarrier, and a pattern encoded microcarrier;
b. the detection microparticles are selected from donor microparticles or acceptor microparticles suitable for light-initiated chemiluminescent detection;
c. the detection particle suitable for multiplex detection of biomolecules further comprises a biocapture substance;
d. the microcarrier with the encoding function is magnetic microcarrier or non-magnetic microcarrier.

3. The detection particle suitable for multiplex detection of biomolecules according to claim 1 or 2, wherein a surface of the microcarrier with the encoding function is covered by a polyelectrolyte shell layer.

4. The detection particle suitable for multiplex detection of biomolecules according to claim 3, wherein the polyelectrolyte shell layer is formed by self-assembly of layers of polycationic electrolyte and polyanionic electrolyte.

5. The detection particle suitable for multiplex detection of biomolecules according to claim 4, wherein the polycationic electrolyte is poly dimethyl diallyl ammonium chloride (PDDA), and the polyanionic electrolyte is poly(sodium 4-styrenesulfonate) (PSS).

6. A biomolecule multiplex detection system for simultaneous detection of multiple biomolecules, which at least comprises a plurality of the detection particles suitable for multiplex detection of biomolecules according to any one of claims 1-5, and matching microparticles, wherein the matching microparticles are light-initiated chemiluminescent microparticles matched with the detection microparticles in the detection particle.

7. The biomolecule multiplex detection system according to claim 6, wherein different to-be-detected biomolecules correspond to different encoded luminescent substance pairs, and the encoded luminescent substance pair is a combination of an encoding substance of the microcarrier with the encoding function and a light-initiated chemiluminescent substance corresponding to the detection microparticle connected to the microcarrier.

8. The biomolecule multiplex detection system according to claim 7, wherein in the biomolecule multiplex detection system, the light-initiated chemiluminescent substance comprises two or more light-initiated chemiluminescent substances with different emission wavelengths and/or excitation wavelengths.

9. The biomolecule multiplex detection system according to claim 6, wherein the system further comprises a fluorescence microscope with a camera or photographic function, and the fluorescence microscope is equipped with at least two excitation light sources, one for excitation of the signal of the microcarrier with the encoding function, and the other for excitation of the light-initiated chemiluminescent signal.

10. The biomolecule multiplex detection system according to claim 9, wherein the system further comprises an image processing device, which is used to match the image obtained after excitation of the signal of the microcarrier with the encoding function with the image obtained after excitation of the light-initiated chemiluminescent signal.

11. A method for preparing the detection particle suitable for multiplex detection of biomolecules according to any one of claims 1-5, comprising the following steps:
mixing the microcarrier with the encoding function and a dispersion containing the detection microparticles, performing a reaction in the dark, to obtain the detection particle suitable for multiplex detection of biomolecules.

12. The method for preparing the detection particle suitable for multiplex detection of biomolecules according to claim 11, further comprising one or more of the following steps:
a. adding the microcarrier with the encoding function dropwise into the dispersion containing the detection microparticles;
b. coupling a biocapture substance on surfaces of the detection microparticles.

13. Use of the detection particle suitable for multiplex detection of biomolecules according to any one of claims 1-5 or the biomolecule multiplex detection system according to any one of claims 6-10 in biomolecules detection.

14. A biomolecule detection method, comprising the following steps:
(1) mixing the detection particle suitable for multiplex detection of biomolecules according to any one of claims 1-5 with a sample solution to be tested, and incubating;
(2) adding matching microparticles, and incubating;
(3) placing the mixture obtained in step (2) in a fluorescence microscope with a camera or photographic function to perform detection, and exciting the signal of the microcarrier with the encoding function and the light-initiated chemiluminescent signal;
(4) collecting the signal of the microcarrier with the encoding function and the light-initiated chemiluminescent signal, respectively, and matching the image obtained after excitation of the signal of the microcarrier with the encoding function with the image obtained after excitation of the light-initiated chemiluminescent signal.

15. The biomolecule detection method according to claim 14, further comprises one or more of the following:
1) in step (1) or (2), performing a washing or washing-free after incubating;
2) in step (4), collecting the signal of the microcarrier with the encoding function and the light-initiated chemiluminescent signal by a camera after passing through a filter set;
3) the microcarrier is selected from magnetic microcarrier and non-magnetic microcarrier;
4) in step (2), the matching microparticle is modified with a biocapture substance;
5) in step (1), adding a biocapture substance.

16. The biomolecule detection method according to claim 15, further comprises one or more of the following:
a. in step (1), when the microcarrier is magnetic microcarrier, incubating after mixing, and performing a magnetic separation and a washing;
b. in step (1) or (2), when the microcarrier is magnetic microcarrier, performing a magnetic separation and a washing after incubating.
